(19) Europäisches Patentamt
European Patent Office
Office européen des brevets

(11) EP 3 278 827 B1

(12) EUROPEAN PATENT SPECIFICATION

(45) Date of publication and mention
of the grant of the patent:
**18.11.2020 Bulletin 2020/47**

(51) Int Cl.:
**A61M 5/20** (2006.01)   **A61M 5/31** (2006.01)

(21) Application number: **16182623.5**

(22) Date of filing: **03.08.2016**

(54) **DEVICE FOR ATTACHMENT TO A PORTABLE LIQUID INJECTION DEVICE**

VORRICHTUNG ZUR BEFESTIGUNG AN EINER TRAGBAREN
FLÜSSIGKEITSABGABEVORRICHTUNG

DISPOSITIF POUR FIXATION A UN DISPOSITIF PORTABLE D'INJECTION DE LIQUIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**07.02.2018 Bulletin 2018/06**

(73) Proprietor: **Valtronic Technologies (Holding) SA
1343 Les Charbonnières (CH)**

(72) Inventor: **LEPPLE-WIENHUES, Albrecht
25300 Pontarlier (FR)**

(74) Representative: **Patentanwälte
Ruff, Wilhelm, Beier, Dauster & Partner mbB
Kronenstraße 30
70174 Stuttgart (DE)**

(56) References cited:
**WO-A1-2010/041260       WO-A1-2016/036574
US-A1- 2002 096 543       US-A1- 2013 310 756**

## Description

FIELD OF THE INVENTION

This invention relates to a device for attachment to a portable liquid injection device. In particular, the inventive device is for attachment to a so-called insulin pen.

Further, the invention relates to a portable liquid injection device, in particular an insulin pen, comprising the inventive device.

BACKGROUND TO THE INVENTION

Many drugs must be administered parenterally, because they are either not resorbed sufficiently or destroyed in the gastrointestinal tract. Typical examples include peptide drugs like insulin. Therefore, in the following the invention is described in the context of an insulin pen, although it extends to other drug delivery devices and drugs.

The pharmacological half-life of these drugs often is very short, leaving the need for multiple daily injections. Therefore, often the injections are performed by the patient himself instead of trained medical professionals. An insulin-dependent diabetic patient e.g. needs to inject multiple times a day at variable doses, adjusted to food intake and physical activity.

To comfort these frequent variable dose self-administrations drug delivery devices have been introduced that are easy to use and typically comprise a reservoir with a plunger, a hypodermic needle and a dosing mechanism. A typical example for such devices is called an "insulin pen". These pens are critical medical devices for the reason that both an overdose and underdose can lead to severe health problems including death. Therefore, those devices are mostly kept very reliable and independent of e.g. battery charge. Most insulin pens on the market today have a strictly mechanic dosing mechanism.

While these injection pens facilitate the multiple injections required, it is difficult and cumbersome for the patient to keep records of all parameters determining the insulin dose adjustment required. Even keeping track of the injection time and dose itself becomes a demanding task, since the typical drug delivery devices do not include means for data communication nor memory. Therefore, it is not rare that a patient does not remember dose, time or the sheer fact of injection in daily life. Severe effects of overdosing or omitted administrations are therefore not rare events and threaten health and life of these patients.

Further, a patient may use one, two or even three different pens during the day. A typical example for a dosage pattern is as follows: In the morning the patent injects a dose of long-lasting insulin with his/her first pen. During the day he/she further injects multiple doses of rapid acting insulin before each meal using a different pen. Timing and doses are typically similar from day to day for the same patient. Deviation from this pattern would be dangerous. The wrong pen/insulin type could be used or the wrong dose injected, or the injection either forgotten or doubled.

In the prior art, DE-A1-10 2004 040 441 discloses a method for determining the filling level of a substance in an ampoule comprising at least two electrodes, whereby the filling level is determined by measuring the capacity of at least one condenser which is formed by said at least two electrodes. According to this publication, the electrodes are located directly in an injection device containing the ampoule, or, preferably, the electrodes are located on the ampoule, in particular are integrated into the ampoule.

As a consequence, the disclosure of DE-A1-10 2004 040 441 requires an integration of electronics into the injection device, making the device more complex and failure prone. For example, the function of the injection device will depend on the availability of a charging current, or a charged power source, and, therefore, will be less reliable than a classical injection device (e.g. insulin pen) with a mechanical dosing mechanism.

WO-A1-2013138830 describes a capacitive NFC (Near Field Communication)-based fill-level sensor for insulin pens. This publication is based on the same technical principle for determining the filling level of the insulin in an ampoule as shown in DE-A1-10 004 040 441. For this purpose, at least two electrodes are located on the ampoule or directly inside of the part of the pen holding the ampoule.

The invention disclosed in WO-A1-2013138830 is inter alia defined by the presence of two antennas allowing the transmittance of measured values to an external data communications unit via NFC.

US-A1-20140018733 discloses a replaceable cap for a transdermal liquid dosing device such as an insulin pen. The cap body also includes a cavity opening into the interior of the cap body and housing a control unit which includes a timer unit, a switch mechanism and a timer display unit. This specific design of the interior of the cap body (control unit including timer unit, switch mechanism and timer display unit) is supposed to help the user determining whether a given dose has been administered. US-A-20140018733 is silent with respect to the applied quantity of the corresponding substance and to the (remaining) filling level of the substance in the dosing device.

US-A1-20020096543 discloses a portable control device to be mounted externally on a conventional injection pen cap containing a touchless proximity sensor that is located on one side of the cap to detect presence/removal either of the metal needle, the insulin ampoule or an electronic tag implemented in the pen. The control device also includes a transmitter that communicates with a console to control a system of colored lights as feedback and reminder for the patient. The disclosed embodiments are complex to use and learn and may require discarding or adapting the existing insulin delivery system already in use by the patient. This disclosure is not suitable to achieve precise, sensitive and reproducible readout of the insulin ampoule content required for tracking of the applied dose, because it does not apply an electric field through the entirety of the insulin container homogenously and also does not comprise means to adjust for inhomogeneity or disturbances of said electric field.

OBJECT OF THE INVENTION

The present invention has the object to overcome the described limitations of the discussed prior art. The invention should create the possibility for any user of an injection device, in particular of an insulin pen, to easily determine the filling state of the drug reservoir of the injection device without a complicated construction of this device and without changing the use of existing devices. In particular, it should be possible to determine the filling level of the drug reservoirs of injection devices from different providers with different constructions.

SUMMARY OF THE INVENTION

The present invention provides a device for attachment (attachment device) to a portable liquid injection device, wherein said device is designed to enclose the drug reservoir of the injection device completely.

In this context the present invention provides a device with the features as disclosed in claim 1 and a portable liquid injection device with the features according to claim 14. Preferred embodiments of the inventive device according to claim 1 are defined in the claims dependent from claim 1. The features of all independent claims and all dependent claims are herewith introduced into the description by reference.

According to the invention the device for attachment to a portable liquid injection device as mentioned above has at least one waveguide enabling an electromagnetic wave to be traveling along at least one axis of the drug reservoir. Said electromagnetic wave is a radio wave.

Said waveguide comprises a conductive surface, in particular a cylindrical conductive surface. Preferably, the axis of said surface is provided to be parallel with an axis of the injection device, in particular parallel with the longitudinal axis of an insulin pen.

The inventive device comprises at least one antenna allowing to transmit an electromagnetic wave into said waveguide and to measure real and imaginary components of transmission/reflectance of said wave.

Preferably, said inventive device has at least one cylindrical conductive surface enclosing the drug reservoir and acting as an electromagnetic waveguide and at least one antenna to emit and to receive electromagnetic waves to be transmitted and or reflected through a part, in particular the entire volume, of the drug reservoir of the injection device.

In the context of the present invention the electromagnetic wave to be used is a radio wave. Radio waves are a type of electromagnetic radiation with wavelengths in the electromagnetic spectrum lower than infrared light. Therefore, radio waves can be defined to have frequencies as high as 300 GHz to as low as several kHz. Another definition of the International Telecommunications Union (ITU) defines radio waves as electromagnetic waves of frequencies lower than 3000 GHz, propagated in space without artificial guide. Radio waves are generated by radio transmitters and received by radio receivers.

Regularly, electromagnetic waves including radio waves are propagating along waveguides. Said waves have been constrained, typically within a hollow conductive (e.g. metallic) tube. The constraining boundaries of the tube prevent the electromagnetic wave from spreading out and thereby reducing in intensity.

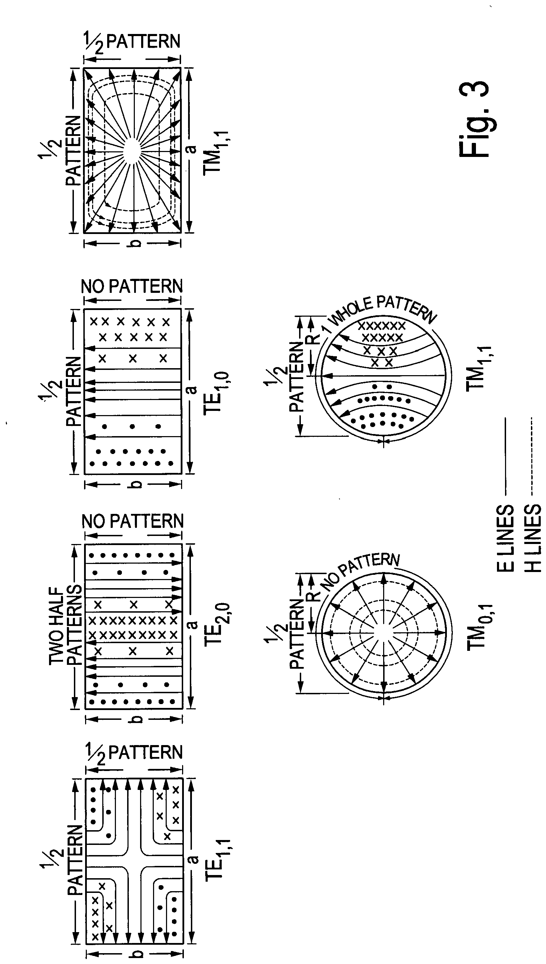

Further, during that process there are a number of different types of electromagnetic waves that can propagate within the waveguide. These different types of waves correspond to the different elements within an electromagnetic wave. Therefore, TE waves are transverse electric waves, also called H waves, being characterized by the fact that the electric vector (E) is always perpendicular to the direction of propagation. Also, TM waves are transverse magnetic waves, also called E waves being characterized by the fact that the magnetic vector (H vector) is always perpendicular to the direction of propagation. These explanations relating to the TE waves and TM waves are to be considered in context with Fig. 3.

In the context of TE and TM waves it is often referred to integers after them: $TE_{m,n}$. These integers indicate the wave modes within the waveguide. Only a limited number of different m, n modes can be propagated along a waveguide dependent upon the waveguide dimensions and format.

Further, for each mode there is a definite lower frequency limit. This is known as the cut-off frequency. Below this frequency no signals can propagate along the waveguide.

The term "conductive surface" means that the surface, irrespective of its actual shape or design, has a low height or thickness compared to the dimensions of the device. Preferably, said surface has the shape of a cylindrical surface with a low height or thickness. Normally, said conductive surface is a metallic surface. The surface can e.g. be the "inner surface" of a separate component of the device, in particular a sleeve or another component integrated into the device. Nevertheless, it is also possible that said conductive surface can be a coating in e.g. an inner part of the device, preferably a metallic coating enabling the electromagnetic wave to be traveling along the axis of the drug reservoir.

In particular, the portable liquid injection device is a so-called insulin pen, i.e. a portable liquid injection device which is used to inject insulin for the treatment of diabetes.

The electromagnetic wave will pass through the entire volume of the drug reservoir of the injection device as evenly and homogenously as practical. It is of special importance that the entire volume of the drug reservoir is passed by said electromagnetic wave, because any parts of the volume not penetrated by the wave will not be measured precisely. Said electromagnetic wave will preferably be used for determining the filling level of a substance in the drug reservoir, in particular of a hormone, e.g. insulin (or a solution containing said substance, respectively).

The transmittance and/or reflectance of the wave guide enclosing the drug reservoir will be measured. This includes the real and the imaginary parts of the wave. The transmittance and / or reflectance changes as well as phase shifts which can be measured can be quite small, e.g. preferably in the sub-picoSiemens to sub-FemtoSiemens range. Further, the signal to noise ratio will be quite small due to unavoidable thermal noise in the wave guide as well as in the measurement electronics. Therefore, this disclosure describes means to reliably, repeatedly and precisely measure those tiny electrical parameters avoiding errors that could affect the determination of the filling level in the drug reservoir down to a statistical error of preferably less than 10 μl, or less than 5 μl, 1 μl, or even 0,5 μl.

In contrast to the methods provided by the prior art, a (separate) device for attachment to a portable liquid injection device is used for determining the filling level of the drug reservoir. In one embodiment this separate device can have the form of a protective cap, replacing the standard protective cap that comes with the device. Therefore, construction and use of the injection device itself need not to be changed for determining the filling level of the drug reservoir. No separate device needs to be added to the pen or its protective cap avoiding the requirement to deal with an additional device. Rather the device will replace the protective cap that already belongs to and comes with the pen. Separate instructions or a separate training for understanding or using the injection device are not necessary. Only the inventive device is used for providing the user of the injection device (patient or health professional) with a complete logged history of dose and time of past injections without any user input required.

It is known that the dielectric constant of water at room temperature is approximately 80 times higher than that of air, and at least an order of magnitude higher than that of glass or plastic. Therefore, the transmission/reflectance wave guide properties follow closely the volume of water in the drug container. The needle can remain on the pen reservoir for the transmission/reflectance measurement.

It is preferred according to the invention that the inventive device is designed in a way that the cylindric conductive surface encloses the elongated, cylindric drug reservoir of the injection device. The drug reservoir typically comprises a glass ampoule with a moveable elastic plunger. The cylindric conductive surface also encloses at least one antenna, located on either one or on both sides of the drug container. Therefore, the electromagnetic wave can be emitted on one side of the drug container, and, depending on wavelength, can either be transmitted by the wave guide including the drug container to a receiving antenna on the other side of the drug container, or can be reflected in the waveguide and the reflected wave can be received by either the same or a second antenna.

Fundamental Information about the involved technology can be found in: The Electromagnetic Theory of Coaxial Transmission Lines and Cylindral Shields S. A. Schelkunoff Bell System Technical Journal. Volume 13, Issue 4, pages 532-579, October 1934

In a preferred embodiment one end of the cylindrical waveguide is excited with a predefined TE1 mode port boundary condition and the other end is terminated with a passive port boundary condition for the same mode. The TE1,1 mode cutoff-frequency of a circular waveguide with radius of 20 mm is approximately 4.4 GHz, which is calculated by

$$f_{c_{ml}} = \frac{c_0 p'_{nm}}{2\pi a}$$

where $c_0$ is the speed of light, $p'_{nm}$ are the roots of the derivative of the Bessel functions $Jn(x)$, m and n are the mode indices, and a is the radius of a waveguide.

In additional preferred embodiments other diameters, especially between 10 and 25 mm, other frequencies, especially between 50 MHz and 50 GHz, propagation modes, and boundary conditions alone or in combinations are implemented.

In particular, a frequency below the cutoff frequency for a given waveguide diameter and propagation mode may be used in a way, that reflection/transmission will be a function of the water column length inside the ampoule and / or that an evanescent wave will be altered by the water column length inside the ampoule.

In another preferred embodiment, a frequency above the cutoff frequency for a given waveguide diameter and propagation mode may be used in a way, that reflection/transmission will be a function of the water column length inside the ampoule, and that measurement of the virtual and real components of a transmitted and/ or reflected wave permits the determination of said water column length.

In another preferred embodiment, variable frequency waves may be used in order to determine the resonance frequency of the cylindrical cavity being formed by the wave guide and enclosing the drug ampoule, again permitting the determination of said water column length.

As a consequence, in the present invention it is not required that the antennas and waveguide surfaces are mechanically very accurately positioned and oriented with respect to the drug reservoir. In other words: With the inventive device, rotational, axial and lateral tolerances are acceptable when the inventive device is attached to the portable liquid injection device. Surprisingly, it is not required in the present invention that the antennas are located close to the drug container, or that the antennas are at a fixed distance to the drug container, or that the transmission/reflectance electromagnetic wave is focused almost exclusively through the drug container. Instead, a relatively large volume of the electromagnetic wave is bypassing the drug container, and large tolerances of distances of the electrodes to the drug container are permitted. Surprisingly, the measurement of transmittance and/or reflectance in the present invention is precise and repeatable enough to measure the liquid volume down to 1 μl, preferably to 0.1 μl precision. In a typical insulin pen at a concentration of 0.1 U/μl, this translates to a precision of 0.1 to 0.01 U (with U being the international unit for the amount of a substance, as known to a person skilled in the art).

As described above, the inventive device is a (separate) device for attachment to a portable liquid injection device, in particular for attachment to a so-called insulin pen. As a consequence, the inventive device can be e.g. in the form of a sleeve which is attached to the injection device for enclosing the drug reservoir of the injection device completely. The sleeve can be pushed onto the insulin pen and positioned on the part of the pen which holds the drug reservoir, e.g. for insulin.

In preferred embodiments of the present invention, the inventive device is in the form of a cap, in particular in the form of a cap of an insulin pen. The term "cap" means any (separate) component to be attached to an injection device, preferably an insulin pen, which covers (and normally protects) another critical component or part of the injection device, e.g. the needle for injecting the corresponding substance. With an insulin pen, a cap normally covers the needle as well as at least a part of the drug reservoir of the pen. This aspect of the invention will be explained in more detail later.

According to further preferred embodiments of the invention, the inventive device is replaceable. This means that the device can reversibly be attached to the injection device, i.e. the device can be removed from the injection device and attached to the injection device again and again, if necessary.

Further, it is preferred that the inventive device has an outer electrical shield located close to the outer surface of the device. Such shield acts as a Faraday cage shielding the wave guide from external interference and avoiding any transmission/reflectance changes caused by factors located outside of the device.

Further, the inventive device comprises an electric circuitry to which inter alia the antennas are connected. The inventive device, in particular the electric circuitry, comprises at least one battery (non-rechargeable or rechargeable). Also other power sources are possible according to the present invention.

Further, according to the invention, a transmission/reflectance measurement circuitry can be provided to the inventive device. With this circuitry, the transmission/reflectance measurement is performed, preferably using a sine or rectangular AC stimulation at a frequency of 2 MHz to 10 GHz and a peak to peak voltage of 0.2 to 30 V (Volt).

The transmission/reflectance measurement is preferably measuring a signal exclusively at the desired frequency including real and imaginary parts.

Further, the inventive device preferably comprises a flexible printed circuit board (PCB). Such PCB can be easily integrated into the inventive device as a substrate for all necessary equipment, e.g. not only including all circuitry, but also the antennas on such PCB.

As a consequence, it is preferred that said flexible PCB is adapted to a sleeve-like or a cap-like component, wherein preferably said flexible PCB is molded or mounted into said sleeve-like or cap-like component.

In addition, according to the present invention, it is preferred that the device comprises at least one display.

In further preferred embodiments of the invention, the inventive device is designed to substitute at least one existing cap of an existing insulin pen. This means that the inventive device, on the one hand, includes all equipment, including the (preferably cylindrical) conductive surface(s) and the antenna(e), necessary according to the present invention, but, on the other hand, corresponds in all other design elements to the existing cap of the existing insulin pen. In other words: The present invention refers to a facultative add on to existing insulin pens. The inventive device can be produced to fit any insulin pen on the market. It allows the user to handle the pen as before, without interference or changed user procedure. In particular, the approved liquid injection device is not altered, the dosage mechanism and its display are not interfered with, and the handling/usage procedure remains unchanged.

As described above, it is the main aspect of the present invention to determine the filling state of the drug reservoir of the injection device. However, the transmission/reflectance changes detected with the inventive device cannot be used only for determining the filling state of the drug reservoir. Those changes which can be detected according to at least one algorithm in an electronic data processor, can be also used to detect at least one of the following conditions or activities which are typical in the use or handling of an insulin pen, namely:

- injection (corresponding to a change in the filling state of the drug reservoir),

- removal of the inventive device from the liquid injection device, in particular of the cap, (decapping),

- attachment of the inventive device to the liquid injection device, in particular of the cap (recapping),

- check for remaining (liquid) drug in the inventive device, in particular the cap (e.g. check for contamination),

- check for a so-called air shot (priming of the injection device/its needle),

- detection of a decrease in the drug (solution) volume typical for an injection and/or air shot,

- detection of an increase in the drug (solution) volume typical for a re-filling procedure and/or for insertion of a new injection device, in particular pen, into the inventive device, in particular the cap,

- detection of temporary changes in transmission/reflectance typical for external manipulations, e.g. detection of human tissue in the proximity of the inventive device,

- detection of changes in transmission/reflectance correlated to temperature changes.

Further, the inventive device can include at least one of the following features, namely:

- a power on-mechanism activating only upon opening a package enclosing the inventive device,

- a so-called reed contact,

- push buttons to scroll through at least one data base on a corresponding display,

- at least one memory unit,

- at least one timer unit,

- at least one temperature compensation unit,

- at least one USB (Universal Serial Bus) or any other wired communication unit to transfer data to an external computer or mobile calculator,

- at least one wireless communication unit to transfer data to an external computer or mobile calculator, e.g. blue-tooth.

In other preferred embodiments of the present invention, the inventive device is composed of at least two parts connecting together to form the device, in particular the cap. In this context, it is preferred that the inventive device includes two parts.

Preferably at least one cavity is formed between the parts for holding other components of the device, e.g.

for holding a battery, a display or other units like a timer unit.

The inventive device is preferably made of a plastic material, in particular of a plastic material formed by injection molding.

Further, it is preferred that the inventive device includes means for fixing, especially for reversibly fixing the device to another item and, therefore, holding the device, in particular the device attached to the injection device on that other item. Preferably, such means are in the form of a pocket clip.

As already described, the inventive device can include a cavity being a compartment for a battery or another power source. Preferably, such compartment has a cover holding the battery or other power source in place.

As already mentioned, the inventive device can comprise at least one display. Preferably, such display is a liquid crystal display (LCD), in particular a four-digit digital LCD.

Finally, the invention includes a portable liquid injection device, in particular an insulin pen, which comprises the inventive device as described above, wherein preferably said inventive device is attached to the injection device, in particular to the insulin pen.

The inventive device in its various embodiments brings a number of benefits to the users of portable liquid injection devices, in particular insulin pens.

As already explained, a typical example for a patient dosage pattern is as follows:

In the morning the patient injects a dose of long-lasting insulin with his/her first pen. During the day he/she further injects multiple doses of rapid acting insulin before each meal. Timing and doses are typically similar from day to day for the same patient. Deviation from this pattern would be dangerous. The wrong pen could be used, or the wrong dose injected, or the injection either forgotten or doubled. In all those cases of error the inventive device having up-to date data e.g. relating to the filling level of the drug reservoir and ideally having learned the typical daily dose pattern can recognize a deviation and warn the patient. The patient can then contact his doctor for advice, or administer glucose or insulin for correcting the error, thus avoiding a hypoglycemic shock or dangerous hyperglycaemic states. In this case of two different pens with e.g. a long-lasting and a rapid-acting insulin two inventive devices will be used, one on each pen respectively. The two devices can communicate wirelessly in order to receive and survey complete dosage information including dosage information from the other pen. One device could be a master with additional features like displays etc. while the other devices could be variants as a slave device.

In another embodiment the devices can communicate with a third device e.g. a smartphone or a blood glucose meter and the dosage pattern logic including alarm functions can then be also implemented in a third device.

In other preferred embodiments, the inventive device comprises intelligent features, i.e. the device contains a microprocessor and an algorithm that can recognize and automatically learn the typical pattern of time and dosage for a given patient. Alternatively a typical pattern can be programmed into the device as well as alarm thresholds, e.g. by wireless data communication. In addition, the airshot pattern used by this patient (squirting a small amount of insulin into the air before injection to prime and test the hypodermic needle) will be programmable. The device will contain a microprocessor running algorithms that analyze the dosage and time and warn the patient when deviating from his typical dosage pattern using an alarm feature. The device comprises means for acoustical, optical or vibrational warnings and alarms.

As mentioned, frequently a patient uses two or more different injection pens containing at least a long-lasting (pen A) and a rapid acting insulin (pen B) respectively. The inventive device then may comprise means featuring a key lock mechanism. A matching device will remain on the pens and allow only the fitting of cap A to pen A and cap B to pen B, excluding confounding the two pens with different types of insulin. The aforementioned key lock mechanism could comprise a plastic ring or sleeve that remains permanently on the pen and contains geometric features that ensure fitting the correct cap to its respective pen only.

Thus, multiple different devices could be used on different pens with different types of insulin. Wireless communication between these devices can ensure that the right pen is used at the correct time and dose and deviations from the correct dosage and time pattern will trigger alarm.

Further, a biometric identification of the patient can be provided by e.g. a fingerprint sensor on the cap or an additional device to exclude confounding or tampering with data belonging to patient.

Further advantages and features of the invention will become clear from the following description of the drawings in conjunction with the dependent claims. The individual features can be realized either singly or separately in combination in one embodiment of the invention. The drawings merely serve for illustration and better understanding of the invention and are not to be understood as in any way limiting the invention.

The figures schematically show:

Fig. 1    an insulin pen and its cap according to the state of the art,

Fig. 2    the drug reservoir of a portable liquid injection device with a cylindrical conductive surface integrated into the drug reservoir as a wave guide,

Fig. 3    a schematic representation of some transmission modes in cylindrical waveguides, and

Fig. 4    an insulin pen and its cap with keylock mechanism which can be implemented with an inventive device.

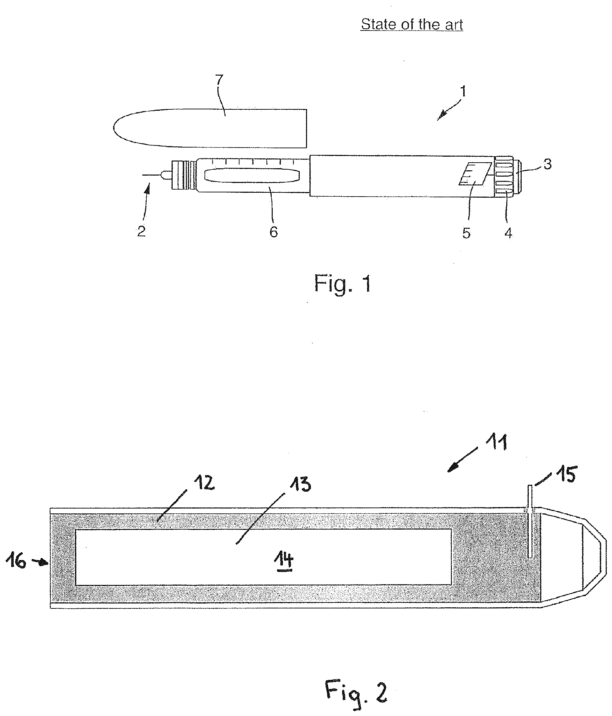

In Fig. 1, a typical insulin pen 1 according to the state of the art is shown. As mentioned earlier, an insulin pen is used to inject insulin for the treatment of diabetes. As is well-known, insulin is a hormone produced by the pancreas. It is important for regulating carbohydrate and fat metabolism in the body.

Insulin pen 1 according to Fig. 1 has a needle 2 at its one end and a button 3 for actuating the injection at the other end. Further, in Fig. 1, a dosage knob 4 and a dose window 5 are shown.

Further, insulin pen 1 has a drug reservoir 6 containing the insulin to be injected into the patient. It is the filling level/filling state of this drug reservoir which shall be monitored according to the present invention.

The insulin pen 1 according to Fig. 1 also has a cap 7 which is used according to the prior art to cover the part of the pen comprising the needle and the drug reservoir.

Fig. 2 shows an embodiment of a liquid injection device 11 (e.g. an insulin pen) in which a wave guide 12 encloses a drug reservoir 13 which is at least partly filled with a drug 14 in liquid form with the drug solution 14 being the bulk of the dielectric material inside the wave guide (12).

According to Fig. 2 the waveguide 12 is in the form of a cylindrical conductive surface lining or coating the inner surface of the cylindrical device 11 and therefore enclosing the drug reservoir 13 completely. As explained earlier, said cylindrical conductive surface forming the waveguide 12 can be provided by a sleeve inserted or integrated into the device, preferably into the cap. The cylindrical conductive surface can also be provided by a coating or even by the inner surface of the device/cap itself. Preferably the conductive surface is made from a metal or a metal alloy.

Further, Fig. 2 shows an antenna 15 protruding into the device. This antenna 15 is coupled to a RF (radio frequency) transceiver (not shown in Fig. 2).

According to a preferred embodiment the radio wave emitted by the antenna 15 travels along the waveguide 12 and is fully reflected at opening 16 of the device 11 because of the impedance change. As a consequence, antenna 15 receives the reflected wave.

In another preferred embodiment, a second antenna (not shown in Fig. 2) is placed close to opening 16 inside the waveguide 12. Therefore, in those embodiments there are two antennas for emitting and/or receiving the radio wave(s).

In another preferred embodiment, only exemplified in this description of Fig. 2, a wavelength of the radio wave (RF wave) is chosen that lies about the cut-off wavelength (frequency) of the waveguide. In another preferred embodiment a wavelength is chosen corresponding to a TMx or TEx transfer mode.

In Fig. 3 some transmission modes in cylindrical waveguides are shown. In correspondence with the above definitions of TE waves and TM waves the electric (E) and magnetic (H) field lines are sketched.

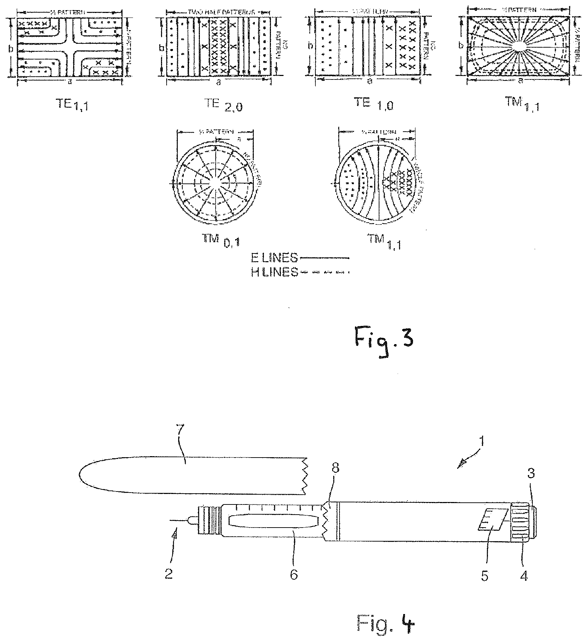

Finally, in Fig. 4 an insulin pen and its cap is shown in which a key-lock mechanism as already described above is implemented. Such a key-lock mechanism comprises two parts which match with each other or fit together like a key and its lock so that e.g. confounding two pens with different types of insulin is excluded. Such a key-lock mechanism can be implemented with all embodiments of the already described inventive device.

For the sake of simplicity the insulin pen and its cap have the same reference numbers as the insulin pen and its cap according to Fig. 1. As a consequence, Fig. 4 shows an insulin pen 1 and its cap 7, wherein insulin pen 1 has a needle 2 at its one end and a button 3 for actuating the injection at the other end. Further, in Fig. 4 a dosage knob 4 and a dose window 5 are shown. The drug reservoir of insulin pen 1 is shown with reference number 6.

According to Fig. 4 cap 7 has a characteristic form at its open end at the right side. This characteristic form 7 matches with the characteristic form of a ring or sleeve 8 which is permanently located on insulin pen 1. Only if the characteristic form of the open end of cap 7 fits into the corresponding characteristic form of sleeve 8 on cap 1, it is possible to attach cap 7 according to Fig. 4 on insulin pen 1 according to Fig. 4. If there is no fit or match, the user of the insulin cap will realize that he or she obviously uses the wrong cap for the corresponding insulin pen.

**Claims**

1. A device for attachment to a portable liquid injection device, in particular for attachment to a so-called insulin pen, wherein said device (11) is designed to enclose the drug reservoir (13) of the injection device completely, and is in the form of a removable sleeve or in the form of a removable cap and wherein said device has at least one wave guide (12) comprising a conductive surface enabling a radio wave to be travelling along an axis of the drug reservoir (13), and an electric circuitry, and wherein at least one antenna (15) is provided allowing to transmit an electromagnetic wave into the wave guide and to measure real and imaginary components of transmission/reflectance of said wave.

2. Device according to claim 1, **characterized in that** the wave guide (12) comprises a cylindrical conductive surface.

3. Device according to claim 1 or claim 2, **characterized in that** the axis of said conductive surface is provided to be parallel with an axis of the injection device, in particular parallel with the longitudinal axis of an insulin pen.

4. Device according to any of the preceding claims, **characterized in that** the device is in the form of a cap of an insulin pen.

5. Device according to any of the preceding claims **characterized in that** the device has an outer electrical shield.

6. Device according to any of the preceding claims, **characterized in that** the device comprises at least one power source, in particular a battery.

7. Device according to any of the preceding claims **characterized in that** the device comprises a transmission/reflectance measurement circuitry.

8. Device according to any of the preceding claims, **characterized in that** the device comprises a flexible printed circuit board (PCB), preferably a flexible PCB wound-up to a cylindrical shape.

9. Device according to claim 8, **characterized in that** said flexible PCB is adapted to a sleeve-like or a cap-like component, wherein preferably said flexible PCB is molded or mounted into said sleeve-like or cap-like component.

10. Device according to any of the preceding claims, **characterized in that** the device comprises at least one display.

11. Device according to any of the preceding claims, **characterized in that** the device is designed to substitute at least one existing cap of an existing insulin pen.

12. Portable liquid injection device, in particular insulin pen, comprising a device according to any of the preceding claims, wherein preferably said device is attached to the injection device, in particular to the insulin pen.

**Patentansprüche**

1. Vorrichtung zum Anbringen an einer tragbaren Flüssigkeitsinjektionsvorrichtung, insbesondere zum Anbringen an einem sogenannten Insulin-Pen, wobei die Vorrichtung (11) so gestaltet ist, dass sie einen Arzneistoffbehälter (13) der Injektionsvorrichtung vollständig umgibt, und in Form einer abnehmbaren Hülse oder in Form einer abnehmbaren Kappe ausgebildet ist, und wobei die Vorrichtung mindestens einen Wellenleiter (12), der eine leitfähige Fläche umfasst, die es ermöglicht, dass eine Funkwelle an einer Achse des Arzneistoffbehälters (13) entlang wandert, und einen elektrischen Schaltkreis aufweist, und wobei mindestens eine Antenne (15) vorgesehen ist, wodurch eine elektromagnetische Welle in den Wellenleiter übertragbar ist und reale und imaginäre Komponenten von Transmissions-/Reflexionsvermögen der Welle gemessen werden können.

2. Vorrichtung nach Anspruch 1, **dadurch gekennzeichnet, dass** der Wellenleiter (12) eine zylinderförmige leitfähige Fläche umfasst.

3. Vorrichtung nach Anspruch 1 oder Anspruch 2, **dadurch gekennzeichnet, dass** die Achse der leitfähigen Fläche parallel zu einer Achse der Injektionsvorrichtung vorgesehen ist, insbesondere parallel zur Längsachse eines Insulin-Pens.

4. Vorrichtung nach einem der vorhergehenden Ansprüche, **dadurch gekennzeichnet, dass** die Vorrichtung in Form einer Kappe eines Insulin-Pens ausgebildet ist.

5. Vorrichtung nach einem der vorhergehenden Ansprüche, **dadurch gekennzeichnet, dass** die Vorrichtung eine äußere elektrische Abschirmung aufweist.

6. Vorrichtung nach einem der vorhergehenden Ansprüche, **dadurch gekennzeichnet, dass** die Vorrichtung mindestens eine Energiequelle, insbesondere eine Batterie, umfasst.

7. Vorrichtung nach einem der vorhergehenden Ansprüche, **dadurch gekennzeichnet, dass** die Vorrichtung einen Messkreis für Transmissions-/Reflexionsvermögen umfasst.

8. Vorrichtung nach einem der vorhergehenden Ansprüche, **dadurch gekennzeichnet, dass** die Vorrichtung eine flexible gedruckte Leiterplatte (PCB), bevorzugt eine in zylindrischer Form aufgewickelte flexible PCB, umfasst.

9. Vorrichtung nach Anspruch 8, **dadurch gekennzeichnet, dass** die flexible PCB auf ein hülsenartiges oder kappenartiges Bauteil angepasst ist, wobei bevorzugt die flexible PCB an das hülsenartige oder kappenartige Bauteil angeformt oder darin angebracht ist.

10. Vorrichtung nach einem der vorhergehenden Ansprüche, **dadurch gekennzeichnet, dass** die Vorrichtung mindestens eine Anzeigeeinrichtung umfasst.

11. Vorrichtung nach einem der vorhergehenden Ansprüche, **dadurch gekennzeichnet, dass** die Vorrichtung als Ersatz für mindestens eine vorhandene Kappe eines vorhandenen Insulin-Pens gestaltet ist.

12. Tragbare Flüssigkeitsinjektionsvorrichtung, insbesondere Insulin-Pen, umfassend eine Vorrichtung nach einem der vorhergehenden Ansprüche, wobei bevorzugt die Vorrichtung an der Injektionsvorrichtung, insbesondere dem Insulin-Pen, angebracht ist.

## Revendications

1. Dispositif à fixer à un dispositif portable d'injection de liquide, en particulier à fixer à un stylo dit à insuline, ledit dispositif (11) étant conçu pour envelopper entièrement le réservoir (13) de médicament du dispositif d'injection, et se présentant sous la forme d'un fourreau amovible ou sous la forme d'un bouchon amovible et ledit dispositif étant doté d'au moins un guide d'ondes (12) comportant une surface conductrice permettant à une onde radio de progresser le long d'un axe du réservoir (13) de médicament, et d'une circuiterie électrique, et au moins une antenne (15) étant installée, permettant de transmettre une onde électromagnétique jusque dans le guide d'ondes et de mesurer des composantes réelle et imaginaire d'une transmission/réflectance de ladite onde.

2. Dispositif selon la revendication 1, **caractérisé en ce que** le guide d'ondes (12) comporte une surface conductrice cylindrique.

3. Dispositif selon la revendication 1 ou la revendication 2, **caractérisé en ce que** l'axe de ladite surface conductrice est placé de façon à être parallèle à un axe du dispositif d'injection, en particulier parallèle à l'axe longitudinal d'un stylo à insuline.

4. Dispositif selon l'une quelconque des revendications précédentes, **caractérisé en ce que** le dispositif se présente sous la forme d'un bouchon de stylo à insuline.

5. Dispositif selon l'une quelconque des revendications précédentes **caractérisé en ce que** le dispositif est doté d'un blindage électrique extérieur.

6. Dispositif selon l'une quelconque des revendications précédentes, **caractérisé en ce que** le dispositif comporte au moins une source d'énergie, en particulier une batterie.

7. Dispositif selon l'une quelconque des revendications précédentes **caractérisé en ce que** le dispositif comporte une circuiterie de mesure de transmission/réflectance.

**8.** Dispositif selon l'une quelconque des revendications précédentes, **caractérisé en ce que** le dispositif comporte une carte à circuit imprimé (PCB) souple, de préférence une PCB souple enroulée en une forme cylindrique.

**9.** Dispositif selon la revendication 8, **caractérisé en ce que** ladite PCB souple est adaptée à un composant de type fourreau ou de type bouchon, ladite PCB souple étant de préférence moulée ou montée dans ledit composant de type fourreau ou de type bouchon.

**10.** Dispositif selon l'une quelconque des revendications précédentes, **caractérisé en ce que** le dispositif comporte au moins un afficheur.

**11.** Dispositif selon l'une quelconque des revendications précédentes, **caractérisé en ce que** le dispositif est conçu pour remplacer au moins un bouchon existant d'un stylo à insuline existant.

**12.** Dispositif portable d'injection de liquide, en particulier stylo à insuline, comportant un dispositif selon l'une quelconque des revendications précédentes, ledit dispositif étant de préférence fixé au dispositif d'injection, en particulier au stylo à insuline.

State of the art

Fig. 1

Fig. 2

TE$_{1,1}$    TE$_{2,0}$    TE$_{1,0}$    TM$_{1,1}$

TM$_{0,1}$    TM$_{1,1}$

E LINES ————
H LINES — — — —

Fig. 3

Fig. 4

Fig. 3

EP 3 278 827 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 102004040441 A1
- WO 2013138830 A1
- DE 10004040441 A1
- US 20140018733 A1
- US 20140018733 A
- US 20020096543 A1

### Non-patent literature cited in the description

- The Electromagnetic Theory of Coaxial Transmission Lines and Cylindral Shields S. A. *Schelkunoff Bell System Technical Journal,* October 1934, vol. 13 (4), 532-579